# EUROPEAN PATENT SPECIFICATION

(11) **EP 3 615 041 B1**
(45) Date of publication and mention of the grant of the patent: **23.02.2022**
(21) Application number: 18722243.5
(22) Date of filing: 24.04.2018
(51) Int. Cl.: A61K 33/14, A61K 33/42, A61K 33/02, A61K 31/14, A61K 31/198, A61K 31/205, A61K 31/6615, A61K 45/06, A23K 20/24, A23K 20/26, A61K 9/00, A61P 15/06, A61P 43/00, A61K 33/06, A23K 20/00, A23K 20/10, A23K 50/30

(54) **CALCIUM BINDER FOR USE IN PREVENTING STILLBIRTH**
CALCIUMBINDEMITTEL ZUR VERWENDUNG IN DER VERHINDERUNG VON FEHLGEBURTEN
LIANT DE CALCIUM DESTINÉ À ÊTRE UTILISÉ DANS LA PRÉVENTION DE LA MORTINATALITÉ

(30) Priority: 25.04.2017 NL 2018777
(43) Date of publication of application: 04.03.2020
(73) Proprietor: Nutreco IP Assets B.V., 5831 JN Boxmeer (NL)
(72) Inventor: LANGENDIJK, Pieter, 3800 AG Amersfoort (NL)
(74) Representative: EP&C
(86) International application number: PCT/NL2018/050260
(87) International publication number: WO 2018/199748

(56) References cited:
- WO-A1-02/49657
- S.T. LIU ET AL: "Effects of dietary citric acid on performance, digestibility of calcium and phosphorus, milk composition and immunoglobulin in sows during late gestation and lactation", ANIMAL FEED SCIENCE AND TECHNOLOGY, vol. 191, 1 May 2014 (2014-05-01), pages 67-75, XP055488007, AMSTERDAM, NL ISSN: 0377-8401, DOI: 10.1016/j.anifeedsci.2014.01.017
- DATABASE WPI Week 201381 Thomson Scientific, London, GB; AN 2013-T75696 XP002782585, & CN 103 211 100 A (UNIV NORTHEAST AGRIC) 24 July 2013 (2013-07-24)
- J. Derouchey ET AL: "Sow Feed Additives on the Market: Are They Worth it?", The Pig Site, 15 March 2009 (2009-03-15), pages 1-8, XP055438720, Retrieved from the Internet: URL:http://www.thepigsite.com/articles/264 0/sow-feed-additives-on-the-market-are-the y-worth-it/ [retrieved on 2018-01-08]
- HOLLINSHEAD F K ET AL: "Calcium, parathyroid hormone, oxytocin and pH profiles in the whelping bitch", THERIOGENOLOGY, LOS ALTOS, CA, US, vol. 73, no. 9, 1 June 2010 (2010-06-01), pages 1276-1283, XP027050786, ISSN: 0093-691X [retrieved on 2010-02-19]

## Description

### GENERAL FIELD OF THE INVENTION

The present invention in general pertains to the field of maximizing the reproductive performance of animals, in particular mammals, further in particular a swine. The invention in particular pertains to maximizing the performance of animals and their offspring by adapting their nutrition. More specifically, the invention relates to a calcium binder as defined in the claims for use in reducing or preventing stillbirth of neonates of sows.

### BACKGROUND ART

Maximizing the performance of pregnant animals and their offspring has for a long time been a major objective of nutritionists. *Inter alia* feed-processing technologies, amino acid supplementation and increased dietary energy density have been used to address the objective of maximizing this performance.

One important aspect of maximizing reproductive performance is optimizing the health of the pregnant animal and its (unborn) offspring during parturition. In particular, it is believed that this may reduce perinatal mortality of the offspring, such as stillbirth (intrapartum mortality) and neonatal mortality. In particular, stillbirth in piglets is mainly caused by oxygen insufficiency, due to repeated episodes of reduced blood perfusion of the placenta, caused by uterine contractions compressing the placenta, and due to stretch and sometimes rupture of the umbilical cord as the foetus travels through the uterus and the birth canal. The oxygen insufficiency leads to increasing blood lactate levels, a decreasing blood pH, and a reduction in extra-cellular fluid base excess in the foetus, a condition referred to as acidosis. When this condition aggravates, this can result in piglet death. Short term oxygen insufficiency is reversible; however, prolonged oxygen insufficiency may lead to irreversible effects and finally, death, and hence it is understandable that stillbirth occurs mainly in piglets born at the end of the birth order. For the same reasons, stillbirth rate is higher in sows that take long to farrow and therefore, the effect of birth order is aggravated in sows with prolonged parturition. It is commonly believed that one of the main causes for prolonged parturition is a reduced concentration of freely available calcium (ionic Calcium, or iCa) in maternal circulation. Calcium is involved in regulating the force and coordination of uterine contractions, and insufficient iCa in the blood results in poor muscle function, and as a consequence, prolonged parturition. For this reason, calcium supplementation to the diet of pregnant animals is often proposed as a measure to reduce perinatal mortality.

Derouchey et al. disclose in "The pig site" (http://www.thepigsite.com/articles/2640/sow-feed-additives-on-the-market-are-they-worth-it/), p. 1-8, 2009 that altering the dietary electrolyte balance (dEB) reduces blood pH and increases the survivability of piglets.

However, the influence of nutrition on maximizing performance, in particular of reproductive performance, has not been conclusively explored yet.

### OBJECT OF THE INVENTION

It is an object of the invention to increase reproductive performance of a pregnant animal which is a sow, in particular to decrease perinatal mortality of offspring, reduce the number of stillbirths, and thereby improve perinatal survival.

### SUMMARY OF THE INVENTION

Completely contrary to the prior art teaching that it is advantageous to supplement the diet with extra calcium, it was found that the addition of a calcium binder to the diet has a positive effect on perinatal mortality, in particular on a reduction of stillbirth. Without being bound to theory, it is believed that by effectively lowering the amount of available calcium in the animal's diet, the animal's natural capability to release calcium from its mineral reserves (in particular from the bone) might be stimulated.

Thus, it has surprisingly been found that it is advantageous to use a calcium binder such as phosphoric acid for oral administration to improve perinatal survival and to prevent stillbirths in sows. In particular it has been found that the number of stillborn offspring of the pregnant animal and/or the percentage of animals having one or more stillborn offspring can be reduced in this way.

### DEFINITIONS

An animal's *diet* is the habitual nourishment of the animal, including feed (solid and liquid feed) and drinking water.

A *calcium binder* is a compound that prevents calcium ions from being dissolved in water in a concentration of more than 10 mM, in particular more than 1 mM.

A salt is *insoluble* in water if an aqueous solution of the salt contains less than 1 mmol of the salt per liter of water at room temperature.

A *dietary supplement* is a product intended for ingestion, which contains a dietary ingredient intended to add nutritional value to the diet. The product can for example be added to the solid feed of the animals (in which case the supplement is often referred to as a top-dress) or to the drinking water (in which case the supplement is often referred to as a drinking water additive).

*Perinatal mortality* is the total of stillbirths and neonatal deaths.

*Stillbirth* is the phenomenon where offspring is born dead, but has died only shortly before (in particular up to 24-48 hours) or during parturition.

*Neonatal death* is the phenomenon where offspring dies shortly after birth, typically within 10 days after birth.

### DETAILED DESCRIPTION OF THE INVENTION

The present disclosure provides a calcium binder such as phosphoric acid to improve perinatal survival in the offspring of an animal.

The animal is preferably a pregnant animal, more preferably a pregnant farming animal, even more preferably a pregnant monogastric farming animal, and most preferably a pregnant sow. According to the invention, the animal is a pregnant sow. As such, the offspring is piglets.

In an embodiment the calcium binder, such as phosphoric acid, is added to the animal's diet (which also encompasses enriching the diet in certain components by extracting other ones). This was found to be a convenient way to provide for oral administration. The calcium binder, such as phosphoric acid, may be added to the drinking water or feed of the animal. For example, the calcium binder may be contained in a composition which is in the form of a premix, a liquid, a powder, granules, a pellet, a dragee, a tablet, a pill, or a capsule.

In particular, the calcium binder, such as phosphoric acid, may be added to the drinking water of the animal. The advantage thereof is that pregnant animals, even right before parturition, normally show regular or even increased drinking behaviour, whereas they might decrease or even cease the eating of solid feed. Thus, consumption of the calcium binder, such as phosphoric acid, may be continued particularly at the point in time when approaching parturition, which is when the iCa in sows is at its highest, and when the calcium binder is most effective.

In yet another embodiment the calcium binder, such as phosphoric acid, is administered in a period of 0 to 5 days before parturition, in particular at least in a period of 1 to 5 days before parturition (which does not exclude that the calcium binder is also administered earlier or later, for example during parturition).

In a particular embodiment, the calcium binder is an anion that forms a water insoluble salt with calcium ions in aqueous solution. Such an ion may for example be a conjugated base ion.

Suitable calcium binders include any compounds which are capable of binding free calcium the ruminant gastro-intestinal tract whereby the free calcium cannot be absorbed by the animal. Thus, the natural calcium regulating defence mechanism of the animal is triggered. Such compounds include phosphoric acid, oxalic acid, sodium oxalate, phytic acid, a phytate, a clay mineral including zeolite, sodium diethylene acetic acid, ethylene diaminetetraacetic acid (EDTA), the sodium salts of EDTA Na₂EDTA and Na₄EDTA, trisodium nitrilotriacetate monohydrate, trisodium ntrioacetate, pentasodium diethylenetriaminepentaacetate, trisodium N-hydroxyethylethylenediaminetriacetate, citric acid, a citrate, a polyphosphate, a tripolyphosphate, a phosphate, a cellulose phosphate, glutamic acid N,N-diacetic acid (GLDA), a sodium salt of GLDA, a potassium salt of GLDA, methylglycine-N,N-diacetic acid (MGDA), a sodium salt of MGDA, a potassium salt of MGDA, ethylenediamine N,N'-disuccinic acid (EDDS), a sodium salt of EDDS, a potassium salt of EDDS, iminodisuccinic acid (IDS), a sodium salt of IDS, or a potassium salt of IDS, or a calcium-free derivative of any such compounds.

According to the invention, the calcium binder is selected from the group consisting of phosphoric acid, phytic acid, a phytate, a polyphosphate, a tripolyphosphate, a phosphate and a cellulose phosphate.

The calcium binder may advantageously be phosphoric acid or a phosphate, particularly when the calcium binder is added to drinking water. Phosphoric acid has the interesting property that it does not form a water insoluble salt with calcium ions at low pH (i.e., when in solution prior to administration to an animal, it remains in soluble form, even when formulated with a calcium salt such as calcium chloride), whilst it precipitates with calcium ions at physiological pH (i.e., in the GI tract of the animal). The calcium binder is preferably an anion that forms a water insoluble salt with calcium ions at physiological small intestinal pH (e.g., a pH in the range of 6-7.5, preferably 6.5-7.5), but not at acidic pH (e.g., a pH below 6, preferably below 5.5).

The calcium binder may be encapsulated by any appropriate encapsulating material. A compound suitable for encapsulation of calcium binder is a compound selected from the group consisting of a fat, a non-calcium derivative of a fat such as a soap and a stearate, a protein, a polysaccharide, a cellulose and a derivative of any such compound, a gum, a glycol and gelatine.

The addition of a calcium binder to an animal's diet is particularly advantageous when combined with administration of a chloride salt, particularly calcium chloride.

The invention will now be further explained using the following non-limiting example.

### EXAMPLES

### Example 1

A calcium binder (phosphoric acid, PhA)) was added in a concentration of 0.01 mol/liter (corresponding to 1 g/L of phosphoric acid). The diets fed to the sows were the same. The results of the study are indicated here below in table 1.

**Table 1: Effect of phosphoric acid on stillbirth**

| | PhA* | Control |
|---|---|---|
| Number of sows | 25 | 24 |
| Stillborn piglets, # | 1.1 | 1.6 |

| | | |
|---|---|---|
| *PhA = phosphoric acid | | |

In this experiment, stillbirth was reduced from 1.6 to 1.1 piglets per litter. It is believed that binding of dietary calcium in the gut and the inherent effect of reducing the amount of calcium available to the animal prior to parturition, has as a consequence that the animal is triggered to mobilise calcium from its own mineral reserves in the bones. Therefore, when demand for calcium is increased as is the case in parturition, the animal is more prepared to face this demand, and better equipped to maintain blood Ca levels. In the example described above, blood calcium levels were indeed increased by the treatments provided (data not shown).

## Claims

1. A calcium binder selected from the group consisting of phosphoric acid, phytic acid, a phytate, a polyphosphate, a tripolyphosphate, a phosphate and a cellulose phosphate, for use in reducing or preventing stillbirth of neonates of sows.

2. A calcium binder for use according to claim 1, wherein the calcium binder is added to the diet of a pregnant sow.

3. A calcium binder for use according to claim 2, wherein the calcium binder is added to the drinking water or feed of the pregnant sow.

4. A calcium binder for use according to any of the preceding claims, wherein the calcium binder is administered in a period of 0 to 5 days before parturition, in particular at least in a period of 1 to 5 days before parturition.

5. Calcium binder for use according to any of the preceding claims, wherein the calcium binder is an anion that forms a water insoluble salt with calcium ions at a pH in the range of 6-7.5, but not at a pH below 6.

6. Calcium binder for use according to any of the preceding claims, which is phosphoric acid or a phosphate.

7. Calcium binder for use according to any of the preceding claims, which is administered in the drinking water of the pregnant sow.

## Patentansprüche

1. Kalziumbindemittel, ausgewählt aus der Gruppe bestehend aus Phosphorsäure, Phytinsäure, einem Phytat, einem Polyphosphat, einem Tripolyphosphat, einem Phosphat und einem Zellulosephosphat, für die Verwendung zur Verringerung oder Verhinderung von Totgeburten bei Neugeborenen von Säuen.

2. Kalziumbindemittel zur Verwendung nach Anspruch 1, wobei das Kalziumbindemittel dem Diätfutter einer trächtigen Sau zugesetzt wird.

3. Kalziumbindemittel zur Verwendung nach Anspruch 2, wobei das Kalziumbindemittel dem Trinkwasser oder Futter der trächtigen Sau zugesetzt wird.

4. Kalziumbindemittel zur Verwendung nach einem der vorhergehenden Ansprüche, wobei das Kalziumbindemittel in einem Zeitraum von 0 bis 5 Tagen vor dem Gebären, insbesondere mindestens in einem Zeitraum von 1 bis 5 Tagen vor dem Gebären, verabreicht wird.

5. Kalziumbindemittel zur Verwendung nach einem der vorhergehenden Ansprüche, wobei das Kalziumbindemittel ein Anion ist, das mit Kalziumionen bei einem pH-Wert im Bereich von 6-7,5, jedoch nicht bei einem pH-Wert unter 6, ein wasserunlösliches Salz bildet.

6. Kalziumbindemittel zur Verwendung nach einem der vorangehenden Ansprüche, welches Phosphorsäure oder ein Phosphat ist.

7. Kalziumbindemittel zur Verwendung nach einem der vorhergehenden Ansprüche, welches über das Trinkwasser der trächtigen Sau verabreicht wird.

## Revendications

1. Liant de calcium choisi parmi le groupe consistant en un acide phosphorique, un acide phytique, un phytate, un polysphosphate, un tri-polyphosphate, un phosphate et un phosphate de cellulose, pour une utilisation dans la réduction ou la prévention de mortinatalité de nouveau-nés de truies.

2. Liant de calcium selon la revendication 1, dans lequel le liant de calcium est ajouté au régime alimentaire d'une truie en gestation.

3. Liant de calcium selon la revendication 2, dans lequel le liant de calcium est ajouté à l'eau de boisson ou à l'alimentation de la truie en gestation.

4. Liant de calcium pour une utilisation selon l'une quelconque des revendications précédentes, dans lequel le liant de calcium est administré dans une période de 0 à 5 jours avant la parturition, en particulier au moins dans une période de 1 à 5 jours avant la parturition.

5. Liant de calcium pour une utilisation selon l'une quelconque des revendications précédentes, dans lequel le liant de calcium est un anion qui forme un sel insoluble dans l'eau avec des ions calcium à un pH compris entre 6 et 7,5, mais pas à un pH inférieur à 6.

6. Liant de calcium pour une utilisation selon l'une quelconque des revendications précédentes, lequel est un acide phosphorique ou un phosphate.

7. Liant de calcium pour une utilisation selon l'une quelconque des revendications précédentes, lequel est administré dans l'eau de boisson de la truie en gestation.
